# EUROPÄISCHE PATENTANMELDUNG

(11) **EP 4 282 521 A1**
(43) Veröffentlichungstag der Anmeldung: **29.11.2023**
(21) Anmeldenummer: 22175518.4
(22) Anmeldetag: 25.05.2022
(51) Int. Cl.: B01J 19/08, B01J 19/12, C02F 1/30, B01J 19/24

(54) **WENDEL-PHOTOREAKTOR**

(71) Anmelder: Peschl Ultraviolet GmbH, 55130 Mainz (DE)
(72) Erfinder: PESCHL, Alexander, 55130 Mainz (DE)
(74) Vertreter: mepat Patentanwälte

(57) **Zusammenfassung**

Die vorliegende Erfindung stellt einen Wendel-Photoreaktor (1) mit zumindest einem Lampenmodul (10) und mit zumindest einer Rohrwendel (20) bereit, die eine Mehrzahl Rohrwindungen (23, 23a, 23b) zwischen einem Eingangsabschnitt (21) und einem Ausgangsabschnitt (22) aufweist, wobei die zumindest eine Rohrwendel (20) um das zumindest eine Lampenmodul (10) angeordnet ist. Der Wendel-Photoreaktor (1) weist eine Trägervorrichtung (30), die die zumindest eine Rohrwendel (20) trägt, und ein Schutzgehäuse (40) das einen Aufnahmeraum (38') umgibt, auf, in dem die Trägervorrichtung (30) mit der zumindest einen Rohrwendel (20) und das zumindest eine Lampenmodul (10) angeordnet sind, wobei die Trägervorrichtung (30) eine vorbestimmte Positionierung der Rohrwendel (20) in Bezug zu dem zumindest einen Lampenmodul (10) und dem Schutzgehäuse (40) bereitstellt.

## Beschreibung

Die Erfindung betrifft einen Wendel-Photoreaktor zur kontinuierlichen Produktion eines photochemischen Reaktionsprodukts.

Aus dem Stand der Technik ist bekannt, dass bei der Entwicklung von Photoreaktoren verschiedene Faktoren Einfluss auf die durch die photochemische Reaktion erreichbare Ausbeute haben. Der einfachste Ansatz besteht in einem Batch-Reaktor mit einer oder mehreren Tauchlampen und einer Umwälzeinrichtung wie einer Pumpe oder einem Rührer, die dafür sorgen, dass das im Reaktor vorgelegte Reaktionsmedium turbulent bewegt wird. Ein Batch-Reaktor gestattet zwar vergleichsweise einfach eine Prozessüberwachung, die Eignung zur großtechnischen Produktion im industriellen Maßstab ist jedoch begrenzt.

Zum kontinuierlichen Betrieb geeignete Photoreaktoren sind Rohr-Photoreaktoren, die - meist in horizontaler oder vertikaler Richtung aufgebaut - auch im großtechnischen Maßstab eingesetzt werden. Rohr-Photoreaktoren bestehen häufig aus zumindest zwei koaxialen Rohren, wobei sich in dem inneren Rohr eine Strahlungsquelle befindet und das Reaktionsmedium durch den Ringspalt zwischen dem äußeren und dem inneren Rohr entlang der Strahlungsquelle geleitet wird.

Dabei ist das Skalieren eines photochemischen Reaktors zum kontinuierlichen Betrieb vom Labor- zum Pilotmaßstab und insbesondere zum Industriemaßstab oft mit Schwierigkeiten verbunden und wird, mangels adäquater Modelle für die Übertragung photonischer Energie in chemische, meist semiempirisch ausgeführt. D. h., dass bei der Skalierung photochemischer Reaktoren aus dem Labormaßstab in den Produktionsmaßstab meist Änderungen an der Konstruktion vorgenommen werden müssen, die über eine reine Vergrößerung hinausgehen, da es wichtig ist, das Verhältnis von bestrahltem Volumen zum Reaktorvolumen und die auftreffende Photonenflussdichte konstant zu halten. Einflussgrößen, die bei der Vergrößerung der Konstruktion berücksichtigt werden sollten, betreffen u. a. Strömungsverhältnisse im Reaktionsmedium sowie Wegstrecken, Phasengrenzen und Wanddicken, die zur teilweisen Streuung und/oder Absorption der eingesetzten Strahlung führen können.

In DE 102010014712 B3 wird daher ein modularer Photo-Röhrenreaktor vorgeschlagen, um das Skalieren auf Produktionsmaßstab bei optimierter Reaktionsführung kostengünstig und unter geringem Zeitaufwand zu ermöglichen. Dazu weist der Photo-Röhrenreaktor, mit dem fluide Medien photochemisch behandelt werden können, eine zentralaxial angeordnete Bestrahlungseinheit auf. Diese enthält wenigstens eine Strahlungsquelle und ist von einer Reaktorwand koaxial umgeben, die einenends durch ein Kopfstück mit Fluideinlass und anderenends ein Endstück mit Fluidauslass begrenzt wird. Die Reaktorwand besteht je nach Länge der Bestrahlungseinheit aus zwei oder mehr zylindrischen Reaktorsegmenten, wobei benachbarte Reaktorsegmente durch einen Zwischenflansch verbunden sind, dessen Innendurchmesser dem der Reaktorsegmente entspricht, sodass die Breite eines Ringspalts zwischen der Bestrahlungseinheit und der Reaktorwand entlang der Reaktorlänge konstant ist.

Aus dem Gebiet der Photobioreaktoren ist ferner zur Kultivierung phototropher Organismen (z.B. Algen, Cyanobakterien) bekannt, vor allem im Labor- oder Pilotmaßstab eine Abwandlung eines Rohrreaktors einzusetzen, der Schlauch-, Helix- oder Wendel-Photoreaktor genannt wird. Dabei wird ein flexibler transparenter Schlauch schraubenförmig um eine Lichtquelle gewunden, sodass - bei gleicher Länge der Lichtquelle - eine längere Verweilzeit des Reaktionsmediums im bestrahlten Bereich erreicht werden kann als bei einem koaxialen Rohr-Photoreaktor.

Allerdings sind Photobioreaktoren nur bedingt mit chemischen Photoreaktoren vergleichbar, da Photobioreaktoren für ein optimales Wachstum von Mikroorganismen meist mit Bedingungen betrieben werden, die Sonnenlicht, Umgebungsdruck und Raumtemperatur entsprechen. Damit erfüllen Photobioreaktoren meist nicht die Anforderungen zum Einsatz im photochemischen Bereich, in dem die Reaktionsbedingungen häufig sehr deutlich von der Raumtemperatur und Umgebungsdruck abweichen können. Ferner sind bei der Konstruktion photochemischer Reaktoren jeder Größenordnung die Sicherheitsanforderungen zu berücksichtigen, die durch die Verwendung von Zündquellen wie den Strahlungsquellen und der zugehörigen Elektronik entstehen können, wenn das bei der photochemischen Reaktion eingesetzte Reaktionsmedium entflammbare bzw. entzündliche Materialien enthält.

Ein Einsatz von Wendel-Photoreaktoren im großtechnischen Maßstab ist daher bislang nicht bekannt.

Ausgehend von diesem Stand der Technik ist es Aufgabe der vorliegenden Erfindung, einen verbesserten Wendel-Photoreaktor bereitzustellen.

Diese Aufgabe wird durch einen Wendel-Photoreaktor mit den Merkmalen des Anspruchs 1 gelöst.

Bevorzugte Ausführungsformen sind in den Unteransprüchen ausgeführt.

Gemäß einer ersten Ausführungsform des erfindungsgemäßen Wendel-Photoreaktors, der zur kontinuierlichen Produktion eines photochemischen Reaktionsprodukts ausgebildet ist, weist dieser zumindest ein Lampenmodul und zumindest eine Rohrwendel auf. Die Rohrwendel weist eine Mehrzahl Rohrwindungen zwischen einem Eingangsabschnitt und einem Ausgangsabschnitt auf. Ein am Eingangsabschnitt zugeführtes Eduktfluid passiert die Rohrwindungen als Reaktionsmedium, das am Ausgangsabschnitt als Produktfluid abgeführt wird, das das photochemische Reaktionsprodukt enthält oder daraus besteht. Die zumindest eine Rohrwendel ist mit den Rohrwindungen um das zumindest eine Lampenmodul angeordnet, wobei zumindest die Rohrwindungen der Rohrwendel für eine Einsatzstrahlung des Lampenmoduls transparent sind. Unter "Einsatzstrahlung" wird vorliegend elektromagnetische Strahlung einer bestimmten Wellenlänge oder bestimmter Wellenlängen bzw. Wellenlängenbereichen verstanden, die zur Durchführung der photochemischen Reaktion zur Herstellung des jeweiligen photochemischen Reaktionsprodukts geeignet ist. Dabei liegt die Einsatzstrahlung für photochemische Reaktionen häufig im UV-Bereich, kann grundsätzlich aber auch abweichend dazu im sichtbaren Spektralbereich liegen. "Für die Einsatzstrahlung transparent" bedeutet hierbei, dass die Rohrwindungen aus einem Material bestehen und eine Wandstärke aufweisen, die für die Einsatzstrahlung einen Transmissionsgrad von zumindest 75 % bereitstellen. Erfindungsgemäß weist der Wendel-Photoreaktor eine Trägervorrichtung auf, die die zumindest eine Rohrwendel lösbar trägt. Ein Schutzgehäuse des Wendel-Photoreaktors umgibt einen Aufnahmeraum, in dem die Trägervorrichtung mit der zumindest einen Rohrwendel und das zumindest eine Lampenmodul lösbar angeordnet sind. Die Trägervorrichtung ist dazu ausgebildet, eine vorbestimmte Positionierung der Rohrwendel in Bezug zu dem zumindest einen Lampenmodul in dem Schutzgehäuse bereitzustellen.

Da die Reaktionsbedingungen von photochemischen Reaktionen häufig höhere Drücke verlangen und mit erhöhten Temperaturen verbunden sind und das Reaktionsmedium ggf. gefährliche Substanzen enthalten kann, dient das Schutzgehäuse zur Sicherung der Umgebung vor Kontaminationen bzw. Verletzungen durch Leckagen an der Rohrwendel oder deren Anschlüssen. Das Schutzgehäuse ist daher entsprechend druckfest ausgeführt. Ferner kann das Schutzgehäuse zur Abschirmung der Strahlung des Lampenmoduls nach außen dienen, indem das Schutzgehäuse aus einem für die Einsatzstrahlung nicht transparenten Material gefertigt wird, das zudem vorzugsweise inert oder zumindest ausreichend stabil gegenüber der Einsatzstrahlung und den im Wendel-Photoreaktor eingesetzten Chemikalien ist.

Die Trägervorrichtung ermöglicht vorteilhaft eine einfache Montage und Demontage der Rohrwendel in dem Schutzgehäuse separat von der Montage bzw. Demontage des Lampenmoduls. Dadurch werden Installation und Austausch der Rohrwendel zur Wartung oder Anpassung deutlich erleichtert. Der Wartungsaustausch einer Rohrwendel kann erforderlich werden, wenn etwa das Material der Rohrwendel infolge der Einsatzstrahlung brüchig oder z. B. durch Ein- oder Ablagerungen intransparent wird.

Die Rohrwendel kann beispielsweise als transparenter Schlauch aus einem flexiblen (Kunststoff-)Material bestehen. Der transparente Schlauch kann mithilfe der Trägervorrichtung zur Rohrwendel mit den gewünschten Windungen geformt werden. Alternativ kann die Rohrwendel aus einem transparenten starren Glas- oder Kunststoffmaterial mit fest geformten Windungen bestehen, die von der Trägervorrichtung gehalten werden. In beiden Fällen können die Windungen der Rohrwendel einer Kurve folgen, die sich, vorzugsweise mit konstanter Steigung, um den Mantel eines gedachten Kreiszylinders windet. Allerdings können die Windungen der Rohrwendel auch von einem gleichmäßigen Schraubenverlauf abweichen, um etwa die auftreffende Strahlungsmenge/-dichte entlang dem Verlauf der Rohrwendel zu variieren. Demzufolge ist auch denkbar, dass sich die Windungen der Rohrwendel mit variierender Steigung und/oder um den Mantel eines gedachten Kegelstumpfes oder eines anderen Rotationskörpers bzw. eines Körpers mit polygonaler Grundfläche wie beispielsweise eines Prismas oder einer Pyramide winden. Die Windungen können ferner, abweichend zu einem üblichen Schraubenverlauf mit gleichbleibender Drehrichtung, entlang ihres Verlaufs Änderungen der Drehrichtung aufweisen und beispielsweise mäanderförmig entlang der Mittelachse von oben nach unten und um die Mittelachse geführt werden. Grundsätzlich soll unter einer Rohrwendel im Sinne der Erfindung jede Rohrstruktur verstanden werden, deren Verlauf sich entlang und um den Mantel eines gedachten geometrischen Körpers erstreckt, der ein Zylinder, ein Prisma, ein Kegel- oder Pyramidenstumpf sein kann oder aus zwei oder mehr der genannten zusammengesetzt sein kann, sodass die Rohrstruktur das Lampenmodul zumindest in dessen Strahlungsbereich umgibt.

Zur einfachen Anpassung der Rohrwendel an unterschiedliche Lampenmodule zur Änderung der Durchsatzmenge oder der photochemischen Reaktion und zur Optimierung der Ausbeute des Reaktionsprodukts gestattet die Trägervorrichtung die Aufnahme unterschiedlicher Rohrwendeln. Diese können sich nicht nur hinsichtlich Material, sondern auch in Bezug auf Rohrdurchmesser und Wandstärke und Steigung der Wendel unterscheiden. Gegebenenfalls kann die Trägervorrichtung auch zur Aufnahme von Rohrwendeln ausgebildet sein, die unterschiedliche Wendel-Durchmesser aufweisen, um den Abstand der Windungen zum Lampenmodul variieren zu können. Mit der Steigung der Wendel wird die Anzahl der Windungen festgelegt, die eine Rohrwendel entlang dem Strahlungsbereich des Lampenmoduls aufweist. Alle diese Parameter bestimmen zusammen mit der Durchflussgeschwindigkeit die Verweilzeit im Bestrahlungsbereich, die die Ausbeute bzw. den Reaktionsumsatz beeinflusst. In einer vorteilhaften Ausgestaltungsform kann die Rohrwendel in Wendelsegmente unterteilt und diese können untereinander verbunden werden, um eine einfachere Handhabung und Wartung zu ermöglichen. Dabei kann eine Rohrwendel mit veränderlichen Wendel-Durchmessern, die einfach aus Wendelsegmenten mit unterschiedlichen Wendel-Durchmessern zusammengesetzt werden kann, bei veränderlichen Prozesseigenschaften (z.B. Viskosität, Transmission) des Eduktfluids während der Passage der Rohrwendel zur präzisen Abstimmung auf den jeweiligen Prozess von Vorteil sein.

Nach einer weiteren Ausführungsform des erfindungsgemäßen Wendel-Photoreaktors liegt in dem Schutzgehäuse zumindest ein längliches Führungselement parallel zu einer durch das Lampenmodul bzw. durch die Rohrwendel definierten Längsachse vor. Die Trägervorrichtung weist zumindest ein Eingriffselement auf, das an dem länglichen Führungselement in Längsrichtung geführt und in einer für die Rohrwendel vorbestimmten Position angeordnet werden kann. So gibt das längliche Führungselement die Positionierung der Trägervorrichtung mit der Rohrwendel durch das an dem länglichen Führungselement geführte Eingriffselement vor. Die Trägervorrichtung kann ferner zumindest ein Halterungselement aufweisen, das zur Halterung eines Abschnitts der Rohrwendel ausgebildet ist, der z. B. an einer der Rohrwindungen, an dem Eingangsabschnitt und/oder an dem Ausgangsabschnitt vorliegen kann. D. h., dass die Trägervorrichtung auch mehrere Halterungselemente aufweisen kann, die sich unterscheiden können, um unterschiedliche Rohrwendelabschnitte halten zu können. Dabei kann ein solches Halterungselement einstückig mit dem Eingriffselement ausgebildet sein oder mit dem Eingriffselement lösbar oder unlösbar verbunden sein. Weist die Trägervorrichtung mehr als ein längliches Führungselement, beispielsweise zwei oder bevorzugt drei Führungselemente auf, ist es möglich, dass ein Halterungselement über ein oder mehrere Eingriffselement(e) nur mit einem oder mit mehreren der Führungselemente oder jedem Führungselement in Eingriff steht.

Ferner kann bei einem erfindungsgemäßen Wendel-Photoreaktor gemäß einer weiteren Ausführungsform der Aufnahmeraum fluiddicht abgedichtet sein und das Schutzgehäuse einen Gehäuse-Zulaufanschluss und einen Gehäuse-Ablaufanschluss für ein erstes Temperiermedium aufweisen, das für die Einsatzstrahlung des Lampenmoduls transparent ist. Auf diese Weise kann der Aufnahmeraum, in dem das Lampenmodul und die Trägervorrichtung mit der Rohrwendel angeordnet sind, mit dem ersten Temperiermedium gefüllt werden, das bevorzugt ein im Betriebstemperaturbereich flüssiges Temperiermedium ist. Der Betriebstemperaturbereich bezeichnet einen Temperaturbereich um eine vorbestimmte Reaktionstemperatur, die sich in der Rohrwendel zur Durchführung der photochemischen Reaktion einstellt oder durch das Temperiermedium eingestellt wird. In den meisten Fällen kann es sich bei dem Temperiermedium um ein Kühlmedium handeln, um z. B. bei einer exothermen Reaktion eine vorbestimmte Reaktionstemperatur annähernd konstant zu halten. Ferner dient das erste Temperiermedium dazu, eine Wärmeübertragung auf das Lampenmodul zu unterbinden. In einer vorteilhaften Ausführung kann das Temperiermedium durch den Gehäuse-Zulaufanschluss und den Gehäuse-Ablaufanschluss im Kreislauf geführt werden, um aufgenommene Wärme außerhalb des Schutzgehäuses abzugeben.

Als Temperiermedium kann eine Flüssigkeit gewählt werden, die für das gesamte Emissionsspektrum des Lampenmoduls oder nur für einen Bereich, der die Einsatzstrahlung umfasst, transparent ist. Folglich können auch Filterflüssigkeiten oder Filterzusammensetzungen als Temperiermedium eingesetzt werden, die als Cut-Off-Filter (Absorption kurzwelliger Strahlung unterhalb einer bestimmten Wellenlänge) oder Bandbreitenfilter wirken, die nur für Strahlung in einem bestimmten Wellenlängenbereich transparent sind, der die Einsatzstrahlung umfasst. Alternativ zu einer Filterflüssigkeit oder flüssigen Filterzusammensetzung kann das Material der Rohrwendel oder eine darauf aufgebrachte Filterbeschichtung eine entsprechende Filterfunktion bereitstellen.

Eine weitere Ausführungsform des erfindungsgemäßen Wendel-Photoreaktors sieht vor, dass das Schutzgehäuse einen zylindrischen Aufnahmeabschnitt aufweist, der einenends mit einer Kopfplatte abdichtend verbunden ist, an der das zumindest eine Lampenmodul lösbar befestigt ist. Am anderen Ende ist der zylindrische Aufnahmeabschnitt entweder mit einem Gehäuseboden oder mit einer Bodenplatte abdichtend verbunden. Die Bezeichnungen mit "Kopf" und "Boden" beziehen sich auf eine vertikale Ausrichtung des Schutzgehäuses bzw. des Wendel-Photoreaktors, sodass die Kopfplatte die obere Begrenzung des Schutzgehäuses und der Gehäuseboden bzw. die Bodenplatte die untere Begrenzung des Schutzgehäuses darstellen. Eine vertikale Ausrichtung des Wendel-Photoreaktors zum Betrieb ist allerdings nicht zwingend, sodass der Wendel-Photoreaktor auch in horizontaler Ausrichtung betrieben werden kann. Daher dienen die Bezeichnungen "Kopf" und "Boden" in erster Linie der Unterscheidung der beiden Enden und sollen nicht eine räumliche Orientierung des Wendel-Photoreaktors einschränken. Ferner ist zu verstehen, dass der zylindrische Aufnahmeabschnitt nicht auf eine Kreiszylinderform beschränkt sein soll, sondern auch davon abweichend ein Zylinder mit einem elliptischen oder ovalen oder einem eckigen oder abgerundeten polygonalen Querschnitt sein kann.

Nach einer weiteren Ausführungsform des erfindungsgemäßen Wendel-Photoreaktors ist der Gehäuse-Zulaufanschluss für das erste Temperiermedium an dem Gehäuseboden oder benachbart zu der Bodenplatte an dem zylindrischen Aufnahmeabschnitt angeordnet. Der Gehäuse-Ablaufanschluss für das erste Temperiermedium befindet sich benachbart zu der Kopfplatte an dem zylindrischen Aufnahmeabschnitt, sodass das erste Temperiermedium den Aufnahmeraum möglichst komplett, d. h. ohne Kurzschlussströmungen durchströmt. Gehäuse-Zulauf- und Gehäuse-Ablaufanschluss können dazu ferner diametral versetzt am Schutzgehäuse angeordnet sein.

Nach noch einer weiteren Ausführungsform des erfindungsgemäßen Wendel-Photoreaktors kann vorgesehen sein, dass der Eingangsabschnitt und der Ausgangsabschnitt der Rohrwendel auf derselben Seite vorliegen und sich insbesondere in eine Richtung parallel zu einer Längsachse der Rohrwendel erstrecken. Als Längsachse der Rohrwendel wird die Rotationsachse eines gedachten Rotationskörpers wie beispielsweise eines Kreiszylinders definiert, um dessen Mantel die Windungen gewunden sind. Auf diese Weisen können sich der Eingangsabschnitt und der Ausgangsabschnitt entweder durch die Kopfplatte oder durch die Bodenplatte aus dem Schutzgehäuse heraus erstrecken. Die Kopfplatte bzw. Bodenplatte weisen zum Durchtritt des Eingangs- und Ausgangsabschnitts entsprechende abgedichtete Öffnungen auf. Die Anordnung des Eingangs- und Ausgangsabschnitts der Rohrwendel in der Bodenplatte kann besonders vorteilhaft sein, wenn das Lampenmodul an der Kopfplatte befestigt ist, sodass die Montage und der Anschluss von Rohrwendel und Lampenmodul von unterschiedlichen Seiten des Schutzgehäuses erfolgen können. Damit der Eingangsabschnitt und der Ausgangsabschnitt der Rohrwendel auf derselben Seite vorliegen, kann die Rohrwendel als zweigängige Rohrwendel ausgebildet sein, bei der sich an den Eingangsabschnitt erste Windungen eines ersten Windungsgangs bis zu einer Kehrwindung anschließen, von der sich zweite Windungen eines zweiten Windungsgangs bis zu dem Ausgangsabschnitt erstrecken. Die Gewindesteigung ist hierbei so gewählt, dass die zweiten Windungen zwischen den ersten Windungen verlaufen. Alternativ zu einer zweigängigen Rohrwendel kann zur Anordnung des Eingangs- und Ausgangsabschnitts auf derselben Seite vorgesehen sein, dass sich von einem Ende der Windungen eine Rücklaufleitung neben den Windungen zu einem Ausgangsabschnitt erstreckt, der sich somit auf der gleichen Seite wie der Eingangsabschnitt befindet, der direkt an das andere Ende der Windungen anschließt. Um eine Verschattung der Windungen im Bestrahlungsbereich des Lampenmoduls zu vermeiden, kann die Rücklaufleitung vorzugsweise außerhalb der Windungen verlaufen.

Nach einer weiteren Ausführungsform des erfindungsgemäßen Wendel-Photoreaktors weist das Lampenmodul ein Tauchrohr und zumindest eine Lampe auf, die in dem Tauchrohr angeordnet ist. Das Lampenmodul weist einen Tauchrohr-Zulaufanschluss und einen Tauchrohr-Ablaufanschluss auf, die mit einem von dem Tauchrohr begrenzten Tauchrohrinnenraum kommunizieren, sodass der Tauchrohrinnenraum mit einem zweiten Temperiermedium gefüllt bzw. durchströmt werden kann. Das zweite Temperiermedium ist bevorzugt ein flüssiges Kühlmedium, um Wärme von der Lampe zum Schutz vor Überhitzung und zur Verlängerung der Lebensdauer abzuführen. Wird als flüssiges Kühlmedium eine elektrisch nichtleitende Flüssigkeit verwendet, kann dabei auf ein zum Tauchrohr zusätzliches Hüllrohr um die Lampe verzichtet werden. Das elektrisch nichtleitende Kühlmedium kann so für einen effektiveren Wärmeübergang direkt mit der Lampenoberfläche in Kontakt gebracht werden. In einer vorteilhaften Ausführung wird das Kühlmedium durch den Tauchrohr-Zulaufanschluss und den Tauchrohr-Ablaufanschluss im Kreislauf geführt, um aufgenommene Wärme außerhalt des Schutzgehäuses abzugeben. Sowohl Tauchrohr als auch Kühlmedium sind zumindest für die Einsatzstrahlung transparent. D. h., dass das Material des Tauchrohrs für das gesamte Emissionsspektrum des Lampenmoduls oder nur für einen Bereich, der die Einsatzstrahlung umfasst, transparent sein kann. Ebenso kann die als Kühlmedium gewählte Flüssigkeit für das gesamte Emissionsspektrum des Lampenmoduls oder nur für einen Bereich, der die Einsatzstrahlung umfasst, transparent sein. Ferner ist es möglich, dass das Tauchrohr und/oder das Kühlmedium anstelle des ersten Temperiermediums eine Filterfunktion aufweisen können, um beispielsweise kurzwellige Strahlung unterhalb einer bestimmten Wellenlänge zu absorbieren oder nur für Strahlung in einem bestimmten Wellenlängenbereich, der die Einsatzstrahlung umfasst, transparent zu sein.

Ferner kann das Lampenmodul eines erfindungsgemäßen Wendel-Photoreaktors gemäß einer weiteren Ausführungsform ein Kopfteil mit zumindest einem elektrischen Anschlusselement aufweisen, das mit einem elektrischen Anschlusselement der Lampe verbunden ist. Das Kopfteil ist an der Kopfplatte angeordnet, wobei sich der Tauchrohr-Zulaufanschluss und der Tauchrohr-Ablaufanschluss durch das Kopfteil und/oder durch die Kopfplatte erstrecken. Kopfplatte und Kopfteil können separate Bauteile sein, die miteinander verbunden sind, es ist aber auch denkbar, dass die Kopfplatte als integraler Bestandteil des Kopfteils ausgebildet sein kann. Das Kopfteil oder die Kopfplatte oder beide sind dazu ausgebildet, die Lampe und/oder das Tauchrohr abgedichtet zu halten. Hierbei ist umfasst, dass das Kopfteil am Tauchrohr zur Abdichtung des Tauchrohrinnenraums angeordnet ist, während das Tauchrohr von der Kopfplatte zur Positionierung des Lampenmoduls im Schutzgehäuse abgedichtet gehalten wird.

Grundsätzlich kann die im Lampenmodul eines erfindungsgemäßen Wendel-Photoreaktors eingesetzte Lampe eine beliebige Strahlungsquelle sein, die die gewünschte Einsatzstrahlung emittiert. Bekannte UV-Strahlungsquellen sind zum Beispiel Mittel- und Niederdruck-Quecksilberdampflampen. Nach einer weiteren Ausführungsform des erfindungsgemäßen Wendel-Photoreaktors kann bevorzugt eine LED-Lampe auf Grund ihres vergleichsweise geringen Stromverbrauchs, hoher Lebensdauer und der hohen Schaltfestigkeit bei spontan vollem Lichtstrom eingesetzt werden. Die LED-Lampe weist eine Mehrzahl von Licht emittierenden Halbleiter-Bauelementen (LEDs) auf, die an einem Trägerkörper über dessen Mantelfläche verteilt angeordnet sind. Der Trägerkörper kann vorzugsweise als Kühlkörper aus einem wärmeleitenden Material wie z. B. Aluminium gefertigt sein. Durch den Trägerkörper erstreckt sich ein Fluidkanal, der an einem kopfseitigen Ende des Trägerkörpers mit dem Tauchrohr-Zulaufanschluss verbunden ist. Am anderen, bodenseitigen Ende des Trägerkörpers mündet der Fluidkanal durch eine Zulauföffnung in den Tauchrohrinnenraum, der über eine Ablauföffnung, die benachbart zu dem kopfseitigen Ende des Trägerkörpers in dem Kopfteil bzw. der Kopfplatte vorliegt, mit dem Tauchrohr-Ablaufanschluss in fluider Verbindung steht. So kann das über den Tauchrohr-Zulaufanschluss zugeführte elektrisch nichtleitende Kühlmedium am bodenseitigen Ende des Trägerkörpers in den Tauchrohrinnenraum austreten, entlang der Manteloberfläche des Trägerkörpers mit den LEDs zum kopfseitigen Ende strömen und über die Ablauföffnung durch den Tauchrohr-Ablaufanschluss ausgeführt werden.

Auf diese Weise kann das elektrisch nichtleitende flüssige Kühlmedium nicht nur bei der Passage des Fluidkanals Wärme aufnehmen, die von den LEDs erzeugt und über den Trägerkörper abgeleitet wird, sondern nimmt auch Wärme durch den direkten Kontakt mit den LEDs beim Durchströmen des Tauchrohrinnenraums auf. Durch diese effektive Wärmeableitung werden Temperaturspitzen vermieden, die in Abhängigkeit der Anordnung und der Leistung der LEDs im Betrieb durchaus auftreten und zur Verkürzung der Lebensdauer der LEDs führen können. Daher können die LEDs bei Nennleistung mit hohen Strömen betrieben werden, um eine hohe Lichtausbeute bzw. Strahlungsintensität zu erreichen, die in Photoreaktoren für chemische Synthesen notwendig ist. Neben der verbesserten Kühlung der LEDs stellt das flüssige Kühlmedium zudem vorteilhaft eine erhöhte Gesamtlicht- bzw. Strahlungsleistung bereit, da auf Grund des Brechungsindex' des flüssigen Kühlmediums die Photonen-Auskopplungseffizienz an der Phasengrenze Diodenoberfläche-Tauchrohrinnenraum vergrößert und die Reflexion an der Phasengrenze Tauchrohrinnenraum-Tauchrohrwand verringert wird.

Nach einer noch weiteren Ausführungsform des erfindungsgemäßen Wendel-Photoreaktors kann vorgesehen sein, dass das im Kühlkörper eingesetzte Kühlmedium sich von dem nichtleitenden Kühlmedium im Tauchrohr unterscheidet, wobei der Fluidkanal im Kühlkörper nicht in den Tauchrohrinnenraum mündet, sondern sich durch den Kühlkörper erstreckt und am kopfseitigen Ende des Trägerkörpers einen separaten Zulauf- und Ablaufanschluss hat. Dann kann die Kühlung des Kühlkörpers mit einem konventionellen Kühlmedium wie Wasser, Ethylenglykol oder anderen Kühlmedien erfolgen, während das Tauchrohr mit der oben beschriebenen nichtleitenden Flüssigkeit aufgefüllt wird, die stationär verbleibt bzw. mit einem getrennten Kühlkreislauf temperiert wird, dessen Tauchrohr-Zulaufanschluss und Tauchrohr-Ablaufanschluss sich von dem separaten Zulauf- und Ablaufanschluss des Kühlkörpers unterscheiden. Dadurch wird die Kühlung der Lampe von der thermischen Abkopplung zum Prozess separiert.

Schließlich sieht eine weitere Ausführungsform des erfindungsgemäßen Wendel-Photoreaktors vor, dass das Schutzgehäuse um eine Schwenkachse, die in einem rechten Winkel zu einer Längsachse des Schutzgehäuses bzw. der Rohrwendel mit dem Lampenmodul verläuft, schwenkbar gelagert ist. Auf diese Weise kann das Schutzgehäuse mit der darin befindlichen Trägervorrichtung, die die eine oder gegebenenfalls mehrere Rohrwendel(n) trägt, und dem Lampenmodul verschwenkt werden, sodass das Schutzgehäuse von einer vertikalen Anordnung in eine horizontale Anordnung - und selbstverständlich auch umgekehrt von horizontal in vertikal - überführt werden kann. Der Wendel-Photoreaktor kann zu diesem Zweck entsprechende Rahmen- bzw. Halterungskonstruktionen mit Gelenkverbindungen als Schwenkvorrichtung aufweisen. So kann der Wendel-Photoreaktor beispielsweise in der vertikalen Anordnung betrieben werden, und in der horizontalen Anordnung können Montage-, Austausch- und Wartungsarbeiten durchgeführt werden, oder umgekehrt. Dabei soll die Schwenkvorrichtung nicht auf einen Schwenkbereich von 90° zwischen einer einzigen vertikalen Anordnung und einer horizontalen Anordnung des Schutzgehäuses beschränkt sein, sondern sie kann auch andere Schwenkwinkel, z. B. durch Verschwenken um 180° oder 360° gestatten. Es ist beispielsweise möglich, den Wendel-Photoreaktor zu Montage-, Austausch- und Wartungsarbeiten durch Schwenken um 180° quasi auf den Kopf zu stellen. Dabei ist es grundsätzlich auch denkbar, dass der Wendel-Photoreaktor in einer zwischen der Horizontalen und Vertikalen liegenden Orientierung angeordnet werden kann, falls dies für den Betrieb oder die Wartung sinnvoll ist.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine Längsschnittansicht eines photochemischen Wendel-Photoreaktors nach einer erfindungsgemäßen Ausführungsform,
- **Fig. 2**: eine Querschnittansicht des photochemischen Wendel-Photoreaktors aus Fig. 1 entlang Schnittlinie A-A,
- **Fig. 3**: eine Längsschnittansicht eines demontierten photochemischen Wendel-Photoreaktors entsprechend Fig. 1,
- **Fig. 4**: eine Längsschnittansicht eines photochemischen Wendel-Photoreaktors nach einer weiteren erfindungsgemäßen Ausführungsform vor Endmontage,
- **Fig. 5**: eine Längsschnittansicht der Trägervorrichtung des photochemischen Wendel-Photoreaktors aus Fig. 4,
- **Fig. 6**: drei perspektivische Ansichten a-c eines photochemischen Wendel-Photoreaktors, der nach einer weiteren erfindungsgemäßen Ausführungsform zwischen einer vertikalen Anordnung a) und einer horizontalen Anordnung c) schwenkbar ist.

Bei der erfindungsgemäßen Vorrichtung handelt sich um einen photochemischen Wendel-Photoreaktor zur kontinuierlichen Produktion eines photochemischen Reaktionsprodukts im großtechnischen bzw. industriellen Maßstab, der aus dem Labor- oder Pilotmaßstab skaliert werden kann.

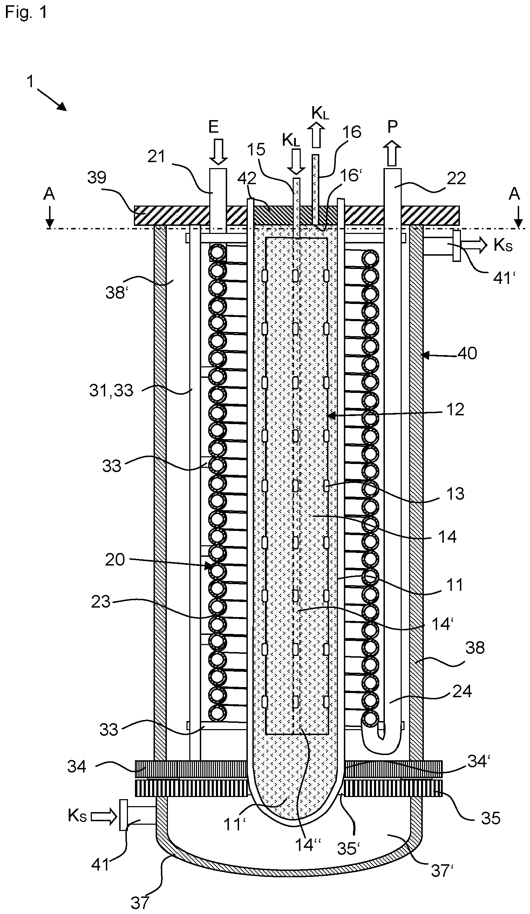

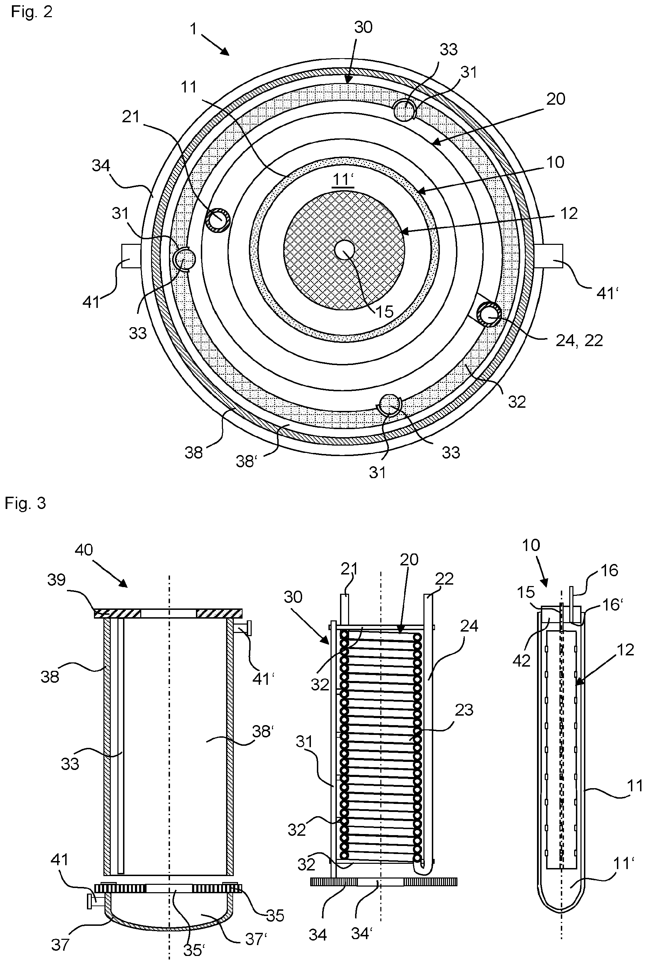

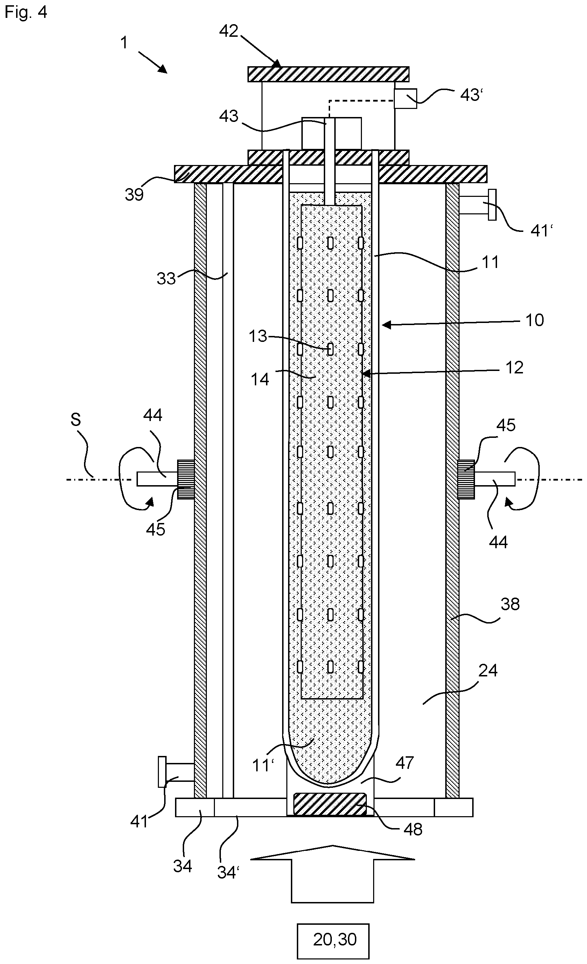

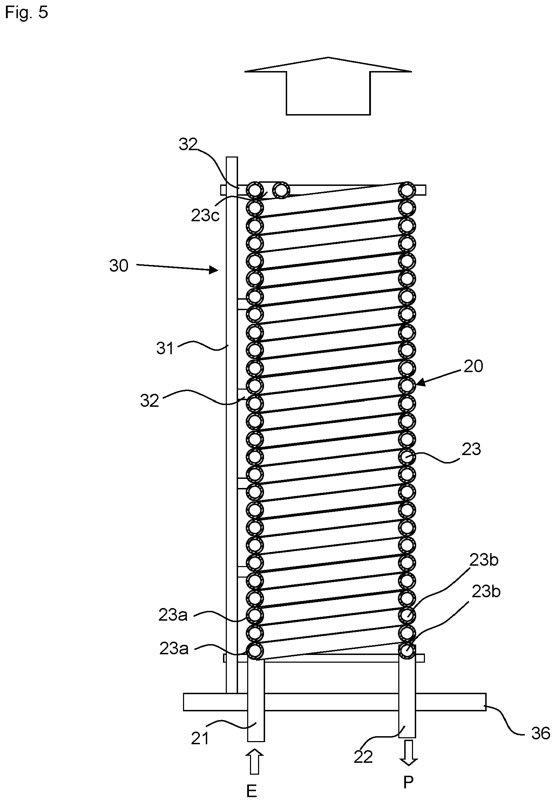

Der in **Fig. 1** **und** **2** dargestellte Wendel-Photoreaktor 1 zur kontinuierlichen Produktion eines Produktfluids P aus einem Eduktfluid E weist als Reaktorkomponenten ein Lampenmodul 10, eine Rohrwendel 20, eine Trägervorrichtung 30 und ein Schutzgehäuse 40 auf, die auch in **Fig. 3** zu sehen sind. **Fig. 4** **und** **5** zeigen ein weiteres Beispiel eines Wendel-Photoreaktors 1, der sich in einigen Details von dem Wendel-Photoreaktor 1 aus **Fig. 1-3** unterscheidet, aber auch aus einem Lampenmodul 10, einer Trägervorrichtung 30 mit einer Rohrwendel 20 und einem Schutzgehäuse 40 zusammengesetzt ist.

Das Schutzgehäuse 40 umgibt einen druckfest abgedichteten Aufnahmeraum 38', in dem das Lampenmodul 10 und die von der Trägervorrichtung 30 gehaltene Rohrwendel 20 angeordnet sind. Das Schutzgehäuse 40 besteht aus einem hier kreiszylindrischen Aufnahmeabschnitt 38, der an einem Ende (kopfseitig) mit einer Kopfplatte 39 abdichtend verbunden ist, an der das Lampenmodul 10 lösbar befestigt ist. Selbstverständlich sind auch von der Kreiszylinderform abweichende Gestaltungen eines Aufnahmeabschnitts möglich. Am anderen Ende (bodenseitig) ist der Aufnahmeabschnitt 38 über einen Basisflansch 34 mit einem Gehäuseboden 37 (**Fig. 1-3**), der dazu einen Bodenflansch 35 aufweist, bzw. mit einer Bodenplatte 36 (**Fig. 5**) mittels einer geeigneten Dichtung abdichtend verbunden bzw. kann abdichtend verbunden werden.

Es wird angemerkt, dass sich die Bezeichnungen "Kopf" und "Boden" auf eine vertikale Anordnung des Schutzgehäuses 40 mit den darin angeordneten Reaktorkomponenten Lampenmodul 10, Rohrwendel 20 und Trägervorrichtung 30 beziehen, wobei sich die Kopfplatte 39 oben und der Gehäuseboden 37 bzw. die Bodenplatte 36 unten befinden. Dies bedeutet allerdings nicht, dass zum Betrieb eine vertikale Anordnung zwingend erforderlich ist, sondern nur, dass es sich dabei um eine bevorzugte, vorteilhafte Anordnung handeln kann. Ein Wendel-Photoreaktor 1 kann ebenso in horizontaler oder anderer Orientierung des Schutzgehäuses 40 mit den darin angeordneten Reaktorkomponenten Lampenmodul 10, Rohrwendel 20 und Trägervorrichtung 30 betrieben werden, wenn dies wünschenswert ist.

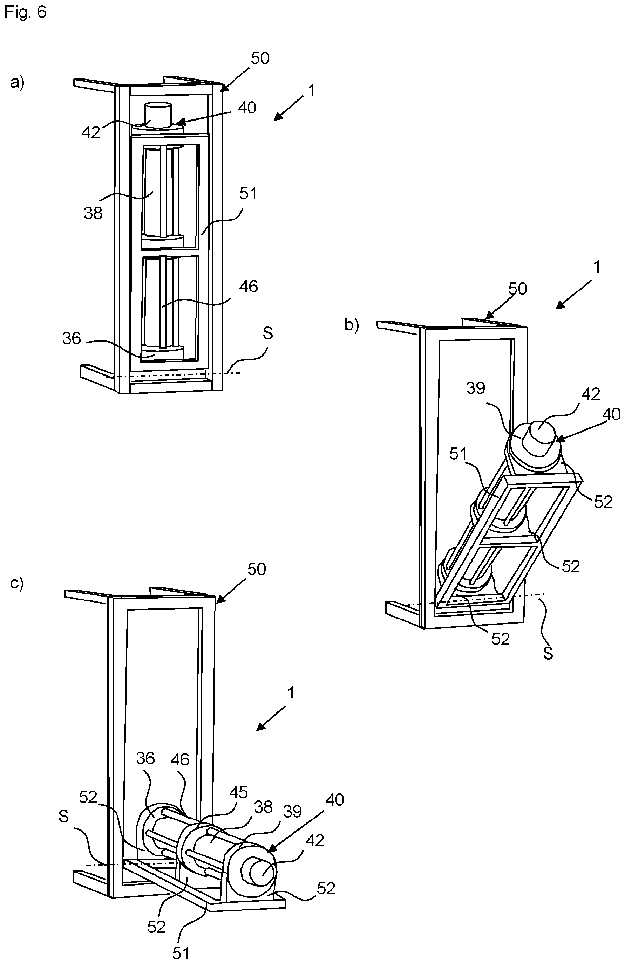

Dabei kann, wie im Beispiel von **Fig. 4** **und** **6** zu sehen ist, der Wendel-Photoreaktor 1 eine Schwenkachse S aufweisen, um die das Schutzgehäuse 40 mit den darin angeordneten Reaktorkomponenten Lampenmodul 10, Rohrwendel 20 und Trägervorrichtung 30 zwischen zumindest einer vertikalen Anordnung und zumindest einer horizontalen Anordnung um 90° bzw. 180°, ggf. um 360°, verschwenkbar ist. Entsprechend verläuft die Schwenkachse S in einem rechten Winkel zu einer Längsachse des Schutzgehäuses 40 bzw. des Lampenmoduls 10 oder der Rohrwendel 20. So kann das Schutzgehäuse 40 mit den darin angeordneten Reaktorkomponenten Lampenmodul 10, Rohrwendel 20 und Trägervorrichtung 30 in einer für den Betrieb gewünschten Orientierung angeordnet werden, die von einer Ausrichtung des Schutzgehäuses 40 zu Wartungs- bzw. Montagezwecken abweichen kann. Das Beispiel in **Fig. 6** zeigt einen Wendel-Photoreaktor 1, dessen Schutzgehäuse 40 mit den darin angeordneten Reaktorkomponenten Lampenmodul, Rohrwendel und Trägervorrichtung aus einer vertikalen Anordnung in **Fig. 6a** durch Verschwenken um die Schwenkachse S (**Fig. 6b**) in eine horizontale Anordnung in **Fig. 6c** überführt werden kann.

Wird der Wendel-Photoreaktor 1 beispielsweise in der vertikalen Anordnung (**Fig. 6a**) betrieben, kann die horizontale Anordnung (**Fig. 6c**) zu Montage- bzw. Wartungszwecken genutzt werden, bei der das Schutzgehäuse 40 sowohl von der Kopfseite als auch der Bodenseite gut zugänglich ist, sodass die Trägervorrichtung mit der Rohrwendel und/oder das Lampenmodul einfach entnommen und ausgewechselt werden können. Selbstverständlich sind davon abweichende Orientierungen des Schutzgehäuses 40 zum Betrieb und zur Wartung/Montage denkbar.

Wie in **Fig. 6** zu sehen, kann der Wendel-Photoreaktor 1 ein Rahmengestell 50 umfassen, das im dargestellten Beispiel das Schutzgehäuse 40 in der vertikalen Anordnung (**Fig. 6a**) umgibt. Das Gestell 50 ist mit einem um die Schwenkachse S schwenkbaren Rahmen 51 verbunden, an dem das Schutzgehäuse 40 befestigt ist. Der Rahmen 51 weist hier dazu drei Befestigungsabschnitte 52 auf, die mit der Kopfplatte 39, der Bodenplatte 36 und einem dazwischen um den Aufnahmeabschnitt 38 angeordnetem Befestigungsring 45 verbunden sind, der über Befestigungsstäbe 46 mit der Kopfplatte 39 und der Bodenplatte 36 verbunden ist. Die Schwenkachse S in diesem Beispiel befindet sich am unteren Ende des Rahmens 51, d. h. im Bereich der Bodenplatte 36. Selbstverständlich kann eine Schwenkvorrichtung zum Schwenken des Schutzgehäuses anders als das nur beispielhaft dargestellte Gestell 50 mit Rahmen 51 ausgeführt sein. Anstelle eines Gestells kann auch ein Behälter mit geschlossenen Wänden eingesetzt werden, die Anordnung der Schwenkachse und Befestigung des Schutzgehäuses können variieren.

Alternativ zum Beispiel aus **Fig. 6** kann die Schwenkachse S, wie in **Fig. 4** dargestellt, in einem mittleren Bereich des Schutzgehäuses 40 vorgesehen sein, sodass vorteilhaft weniger Beschleunigungskräfte auftreten, wenn das Schutzgehäuse 40 mit den darin angeordneten Komponenten verschwenkt wird. Bei der Anordnung von Achselementen 44 an dem mittigen Befestigungsring 45 kann ggf. auf einen Halterahmen 51 verzichtet werden und das Schutzgehäuse 40 direkt in einem entsprechenden Rahmengestell bzw. Außenbehälter gelagert sein. Beide Schwenkvarianten haben Vor- und Nachteile in Bezug auf Zugänglichkeit, Platzbedarf und Beschleunigungskräfte beim Schwenken, sorgen aber beide für eine Erleichterung bei der Montage/Demontage von Lampenmodul und/oder Trägervorrichtung mit Rohrwendel in bzw. aus dem Schutzgehäuse.

Die Rohrwendel 20 des Wendel-Photoreaktors 1 aus **Fig. 1-3** weist eine Mehrzahl Rohrwindungen 23 und an dem kopfseitigen Ende einen Eingangsabschnitt 21 zur Zufuhr eines Eduktfluids E auf. Der Eingangsabschnitt 21 erstreckt sich parallel zu der Längsachse der Rohrwendel 20, die der Rotationsachse eines gedachten Kreiszylinders entspricht, um dessen Mantel die Windungen 23 gewunden sind. Am bodenseitigen Ende sind die Rohrwindungen 23 mit einer Rücklaufleitung 24 verbunden, die sich außenseitig entlang den Windungen 23 parallel zu der Längsachse bis zu einem Ausgangsabschnitt 22 zur Abfuhr des Produktfluids P mit dem Reaktionsprodukt erstreckt. Der Ausgangsabschnitt 22 ist somit auf derselben Seite der Rohrwendel 20 wie der Eingangsabschnitt 21 und parallel dazu angeordnet, sodass Eingangs- und Ausgangsabschnitt 21, 22 auf derselben Seite an jeweils eine entsprechende Edukt- und Produktleitung (nicht dargestellt) angeschlossen werden können. Sowohl der Eingangsabschnitt 21 als auch der Ausgangsabschnitt 22 erstrecken sich hier durch die Kopfplatte 39. Dort sind entsprechende Öffnungen zur Anordnung des Eingangsabschnitts 21 und des Ausgangsabschnitts 22 vorgesehen, die durch geeignete Dichtungen (nicht dargestellt) abgedichtet werden.

Die in **Fig. 5** dargestellte Rohrwendel 20 ist als zweigängige Rohrwendel 20 mit aufsteigenden Windungen 23a, die einen ersten Windungsgang bilden, und absteigenden Windungen 23b, die einen zweiten Windungsgang bilden, ausgeführt, wobei die beiden Windungsgänge kopfseitig durch eine Kehrwindung 23c verbunden sind. So befinden sich sowohl der Eingangsabschnitt 21, der mit der ersten aufsteigenden Windung 23a verbunden ist, als auch der Ausgangsabschnitt 22, der mit der letzten absteigenden Windung 23b verbunden ist, auf derselben Seite der Rohrwendel 20. Auch hier erstrecken sich Eingangs- und Ausgangsabschnitt 21, 22 parallel zu der Längsachse der Rohrwendel 20 und verlaufen in diesem Beispiel durch die Bodenplatte 36 bzw. darin vorgesehene Öffnungen, die entsprechend abgedichtet sind.

Auf Grund der Befestigung des Lampenmoduls 10 an der Kopfplatte 39 ist die Anordnung der Rohrwendel 20 mit dem Eingangs- und Ausgangsabschnitt 21, 22 an der Bodenplatte 36 besonders vorteilhaft, da sich dann die Anschlüsse der Rohrwendel 20 und die Anschlüsse des Lampenmoduls 10 auf gegenüberliegenden Seiten des Schutzgehäuses 40 befinden und die Montage und Demontage vereinfacht wird. Dabei ist diese Anordnung des Eingangs- und Ausgangsabschnitts 21, 22 der Rohrwendel 20 nicht auf die zweigängige Ausführung aus **Fig. 5** beschränkt, sondern kann auch entsprechend für eine Rohrwendel 20 mit Rücklaufleitung 24 wie in **Fig. 1-3** umgesetzt werden. Umgekehrt kann eine zweigängige Rohrwendel ohne Rücklaufabschnitt auch in einem Wendel-Photoreaktor entsprechend **Fig. 1-3** eingesetzt werden, bei dem Eingangs- und Ausgangsabschnitt durch die Kopfplatte verlaufen. Ohne Schwenkbarkeit müsste bei einem vertikal orientierten Schutzgehäuse 40 oberhalb und unterhalb des Schutzgehäuses 40 jeweils ein entsprechender Montageraum freigehalten werden, um eine Montage bzw. Demontage des Lampenmoduls von oben und eine Montage bzw. Demontage der Trägervorrichtung mit der Rohrwendel von unten zu ermöglichen.

Hierbei kann die Schwenkbarkeit sinnvoll eingesetzt werden, indem Montage bzw. Demontage nur von oben stattfinden, auch, wenn nur die Trägervorrichtung 30 mit der Rohrwendel 20 ausgetauscht werden soll. Dann kann das Schutzgehäuse 40 in vertikaler Orientierung quasi auf den Kopf gestellt werden, sodass die Kopfplatte 39, an der das Lampenmodul 10 befestigt ist, nach unten und die Bodenplatte 36, die mit der Trägervorrichtung 30 und der Rohrwendel 20 verbunden ist, nach oben weist. Mit der Bodenplatte 36 kann die Trägervorrichtung 30 mit der Rohrwendel 20 dann nach oben aus dem Aufnahmeraum 38' entfernt werden, während das Lampenmodul 10 im Schutzgehäuse 40 verbleibt. Nach Wechsel der Rohrwendel 20 an der Trägervorrichtung 30 kann diese wieder um das Lampenmodul 10 eingeführt werden, ehe der Wendel-Photoreaktor 1 nach Befestigung der Bodenplatte 36 an dem Aufnahmeabschnitt 38 in seine Betriebsanordnung überführt wird. Die Trägervorrichtung 30 kann dabei fest mit der Bodenplatte 36 verbunden sein, sodass das Einführen und Herausnehmen der Trägervorrichtung 30 mit der Rohrwendel 20 zusammen mit der Bodenplatte 36 erfolgt. Alternativ dazu kann die Trägervorrichtung 30 mit der Rohrwendel 20, wenn dies aus Gewichtsgründen vorteilhafter erscheint, lösbar mit der Bodenplatte 36 verbunden sein. Dann kann bei der Montage zuerst die Trägervorrichtung 30 mit der Rohrwendel 20 eingeschoben und die Bodenplatte 36 danach montiert wird, bzw. bei der Demontage zunächst die Bodenplatte 36 entfernt und dann die Trägervorrichtung 30 mit der Rohrwendel 20 entnommen wird.

Wie in **Fig. 1** zu sehen ist, umgibt die Rohrwendel 20 in dem Wendel-Photoreaktor 1 das Lampenmodul 10, wobei die Rohrwindungen 23 den Bestrahlungsbereich des Lampenmoduls 10 abdecken. Die Rohrwendel 20 ist in **Fig. 5** mit Rohrwindungen 23a, 23b ausgebildet, die bei Anordnung der Rohrwendel 20 im Schutzgehäuse 40 um das Lampenmodul 10, wie in **Fig. 4** durch den Blockpfeil angedeutet, den Bestrahlungsbereich des Lampenmoduls 10 abdecken. Das zugeführte Eduktfluid E wird bei der Passage der Windungen 23, 23a, 23b als Reaktionsmedium der Einsatzstrahlung ausgesetzt, wodurch die photochemische Reaktion zur Herstellung des Reaktionsprodukts ausgelöst wird, sodass aus der Rohrwendel Produktfluid P abgeführt wird. Abhängig von der Steigung, bzw. Anzahl der Windungen 23, 23a, 23b, dem Durchmesser der Windungen und des Rohrs sowie der Länge des gewundenen Rohrabschnitts und dem Volumenstrom des Eduktfluids E können unterschiedliche Verweilzeiten realisiert werden. Der Durchmesser des Rohrs bzw. der Windungen wird anhand der hydrodynamischen Eigenschaften sowie der Absorption ausgewählt.

Die Rohrwindungen 23, 23a, 23b sind daher aus einem für die Einsatzstrahlung des Lampenmoduls 10 transparenten Material, das ein flexibles Kunststoffmaterial oder ein starres Kunststoff- oder Glasmaterial sein kann. In den dargestellten Beispielen sind die Rohrwendeln 20 einstückig ausgebildet, sodass auch die jeweiligen Eingangs- und Ausgangsabschnitte 21, 22 bzw. die Rücklaufleitung 24 aus demselben transparenten Material bestehen wie die Rohrwindungen 23, 23a, 23b. Abweichend dazu kann allerdings auch vorgesehen sein, dass die Windungen 23, 23a, 23b und die Eingangs- und Ausgangsabschnitte 21, 22 bzw. die Rücklaufleitung 24 separat gefertigt sind und zur Bildung einer Rohrwendel 20 miteinander verbunden werden. In einem solchen Fall können die Eingangs- und Ausgangsabschnitte 21, 22 bzw. die Rücklaufleitung 24 auch aus einem anderen Material bestehen, das für die Einsatzstrahlung nicht transparent ist, um eine gute Prozesskontrolle sicherzustellen. Um dies auch bei einstückigen Rohrwendeln 20 sicherzustellen, kann die Rücklaufleitung 24 und ggf. auch der Eingangs- und/oder Ausgangsabschnitt 21, 22 verschattet werden. Dies kann z. B. durch Aufbringung einer die Einsatzstrahlung absorbierenden Beschichtung oder durch die Anordnung entsprechender Abschirmelemente erfolgen.

Die mechanische Integrität der Rohrwendel kann je nach Material problematisch sein, sodass die Rohrwendel ggf. materialbedingt und aus Alterungsgründen nicht als Druckgerät unter der PED (Druckgeräterichtlinie) nach AD 2000 eingestuft werden kann, was zur Durchführung einer photochemischen Reaktion ggf. erforderlich sein kann. In diesem Fall stellt das Schutzgehäuse ein sicheres Containment System (Sicherheitsumschließung) zum Schutz von Personen und der Umgebung im Fall einer Leckage oder des Platzens einer Rohwendel aus Kunststoff oder Glas dar.

Eine einfache Montage und Demontage des Wendel-Photoreaktors 1 wird durch die Trägervorrichtung 30 ermöglicht, die, wie **Fig. 1 bis 3** **und** **5** zu entnehmen ist, diverse Halterungselemente 32 aufweist, die an den Windungen 23, 23a, 23b, an den Eingangs- und Ausgangsabschnitten 21, 22 bzw. der Rücklaufleitung 24 lösbar befestigt sind. Die Halterungselemente 32 sind mit Eingriffselementen 31 verbunden, wobei die beispielhafte Trägervorrichtung 30, wie **Fig. 2** zeigt, drei als längliche Profilelemente ausgebildet Eingriffselemente 31 aufweist, die auf einer gedachten Kreislinie um die Rohrwendel 20 angeordnet sind und sich parallel zur Rotationsachse der Rohrwendel 20 erstrecken, die bei Anordnung im Wendel-Photoreaktor 1 der Längsachse des Lampenmoduls 10 entspricht. Um die korrekte Positionierung der Trägervorrichtung 30 mit der Rohrwendel 20 in dem Schutzgehäuse 40 in Bezug auf das Lampenmodul 10 sicher zu stellen, weist der Wendel-Photoreaktor 1 entsprechend den drei länglichen Eingriffselementen 31 drei längliche Führungselemente 33 auf, die parallel zu einer durch das Lampenmodul 10 definierten Längsachse in dem Schutzgehäuse 40 vorliegen. Mit den Eingriffselementen 31 kann die Trägervorrichtung 30 mit der Rohrwendel 20 an den länglichen Führungselementen 33 in das Schutzgehäuse 40 in korrekter Position in Bezug zu dem Lampenmodul 10 eingeführt werden.

In der vorteilhaften Ausführungsform der Erfindung, die in **Fig. 4** **und** **5** gezeigt ist und die verdeutlicht, dass der Anschluss der Rohrwendel 20 durch die Bodenplatte 36 erfolgt, spielt die Rotationlage der Rohrwendel 20 keine Rolle, sodass die länglichen Führungselemente 33 im Schutzgehäuse 40 und die Eingriffselemente 31 der Trägervorrichtung 30 gleichmäßig verteilt auf der gedachten Kreislinie um die Rohrwendel 20 angeordnet werden können. Für den Fall, dass die Rohrwendel 20 im Schutzgehäuse 40 nur in einer bestimmten Rotationslage um das Lampenmodul 10 montiert werden kann, damit wie im Beispiel von **Fig. 1 bis 3** die Lage des Eingangs- und Ausgangsabschnitts 21, 22 der Rohrwendel 20 mit den dafür vorgesehenen Öffnungen in der Kopfplatte 39 korrespondiert, kann die Anordnung der länglichen Führungselemente 33 im Schutzgehäuse 40 von einer rotationssymmetrischen Anordnung abweichen. Mit entsprechender unsymmetrischer Anordnung der Eingriffselemente 31 kann die Trägervorrichtung 30 nur in einer einzigen Rotationslage eingeführt werden, in der die länglichen Führungselemente 33 mit den Eingriffselementen 31 fluchten.

Die so als Gestell ausgebildete Trägervorrichtung kann unterschiedliche Rohrwendeln aus flexiblem oder starrem Kunststoff oder aus Glas aufnehmen. Entsprechend können die Windungen einer Rohrwendel aus flexiblem Kunststoffmaterial in unterschiedlichen Durchmessern und Längen auf die Trägervorrichtung gewickelt werden, während Rohrwendeln aus starrem Kunststoff oder Glas in die Trägervorrichtung eingesetzt werden können. Durch die einfache Einführung und Entnahme in das und aus dem Schutzgehäuse können Bestückung und Austausch der Trägervorrichtung komfortabel außerhalb des Schutzgehäuses und unabhängig von dem Lampenmodul durchgeführt werden. Ist die Trägervorrichtung mit der Rohrwendel bestückt, wird die Trägervorrichtung mit den Eingriffselementen an den länglichen Führungselementen in das Schutzgehäuse eingeführt und dabei automatisch über das Lampenmodul geschoben, sodass im Betrieb des Wendel-Photoreaktors die Windungen der Rohrwendel von innen durch das Lampenmodul bestrahlt werden.

Modifikationen der Führungselemente und der Eingriffselemente, die von den dargestellten Beispielen bezüglich Anzahl und Ausführung abweichen, sind ohne weiteres möglich und fallen unter den Schutzumfang. So kann ein erfindungsgemäßer Wendel-Photoreaktor mehr oder weniger als drei Führungselemente und drei Eingriffselemente aufweisen, die in abweichender Anordnung im Schutzgehäuse vorliegen können. Anders als dargestellt, ist es auch möglich, dass die Halterungselemente nicht mit einem länglichen Eingriffselement verbunden sind, sondern dass einzelne oder alle Halterungselemente mit separaten Eingriffselementen ausgebildet sind, die mit einem der länglichen Führungselemente im Schutzgehäuse in Eingriff gebracht werden können. Bezüglich der länglichen Führungselemente ist es ferner denkbar, dass diese nicht wie dargestellt als separate Bauteile vorliegen, sondern beispielsweise an der Innenwand des Schutzgehäuses ausgeformt sein können. Und alternativ zur dargestellten Schienenführung, kann das längliche Führungselement beispielsweise ähnlich einer Leitspindel ausgebildet sein, auf denen als Eingriffselemente der Trägervorrichtung beispielsweise Gleitbuchsenelemente aus Kunststoff, z. B. PTFE, geführt werden können.

Das zum Betrieb des Wendel-Photoreaktors 1 abgedichtete Schutzgehäuse 40 dient nicht nur als Sicherheitsumschließung, sondern auch der Temperierung der Rohrwendel 20 bzw. des darin geführten Reaktionsmediums von außen, um eine optimale Temperatur während der photochemischen Reaktion einzustellen. Nach dem Verschließen des Schutzgehäuses 40 wird der Aufnahmeraum 38' mit einem flüssigen Temperiermedium Ks gefüllt, das für die Einsatzstrahlung des Lampenmoduls 10 transparent ist. Dazu weist das Schutzgehäuse 40 einen Gehäuse-Zulaufanschluss 41 auf, der im Beispiel von **Fig. 1 bis 3** an dem Gehäuseboden 37 und im Beispiel von **Fig. 4** **und** **5** an dem zylindrischen Aufnahmeabschnitt 38 benachbart zu der Bodenplatte 36 angeordnet ist. Um für eine bessere Temperaturkontrolle das Temperiermedium Ks im Kreislauf zu führen, weist das Schutzgehäuses 40 ferner einen Gehäuse-Ablaufanschluss 41' auf, der benachbart zu der Kopfplatte 39 an dem zylindrischen Aufnahmeabschnitt 38 und diametral zu dem Gehäuse-Zulaufanschluss 41 angeordnet ist. Zur Kreislaufführung schließen sich (nicht dargestellt) dann entsprechende Kreislaufleitungen, die in bekannter Weise mit einer Pumpe und ggf. einem Wärmetauscher verbunden sind, an den Gehäuse-Zulaufanschluss 41 und den Gehäuse-Ablaufanschluss 41' an.

Beispielhafte Temperiermedien Ks umfassen elektrisch nichtleitende Kühlflüssigkeiten wie z. B. Silikonöle, aber auch einfache Kühlflüssigkeiten wie Wasser und Ethylenglycol. Optional können ferner Filterflüssigkeiten als Temperiermedium Ks verwendet werden, um als Cut-Off-Filterflüssigkeit kurzwellige Strahlung des Lampenmoduls unterhalb einer bestimmten Wellenlänge zu absorbieren oder als Bandbreitenfilterflüssigkeit nur (UV-) Strahlung des Lampenmoduls in einem bestimmten Wellenlängenbereich durchzulassen. Aus dem Stand der Technik sind wässrige Zusammensetzungen für derartige Filterlösungen bekannt, wobei durch Variation der Konzentration und Mischung gelöster Salze (z. B. CuSO₄, Fe₂(SO₄)₃, FeSO₄, FeCl₃, Na₂WO₄, SnCl₂, Na₃VO₄, BiCl₃, KVO₃, KNO₂, K₂CrO₄, NiSO₄, CoSO₄,...) unterschiedliche Filterwellenlängen eingestellt werden können.

Das in dem Wendel-Photoreaktor 1 in **Fig. 1 bis 5** dargestellte Lampenmodul 10 weist ein Tauchrohr 11 auf, das koaxial in der Rohrwendel 20 angeordnet ist. Am kopfseitigen Ende ist das bodenseitig geschlossene Tauchrohr 11 von der Kopfplatte 39 aufgenommen, sodass ein vom Tauchrohr 11 umschlossener Tauchrohrinnenraum 11' von dem - ggf. mit dem Temperiermedium Ks gefüllten - Aufnahmeraum 38' im Schutzgehäuse 40 getrennt ist. Das geschlossene Ende des Tauchrohrs 11 ist im Bodenbereich des Schutzgehäuses 40 gelagert, um das Tauchrohr 11 stabil zu halten. Dazu sind in **Fig. 1 bis 3** entsprechende koaxial ausgebildete Durchtrittsöffnungen 34', 35' in dem Basisflansch 34 und dem Bodenflansch 35 zu sehen, die - anders als in der Darstellung zu sehen - weitere Durchgangsöffnungen aufweisen könnten, damit das durch den Gehäuse-Zulaufanschluss 41 am Bodenabschnitt 37 zugeführte Temperiermedium Ks aus dem Bodenraum 37' in den Aufnahmeraum 38' strömen kann. Das Lampenmodul 10, das in **Fig. 4** gezeigt ist, ist an dem geschlossenen Ende des Tauchrohrs 11 in einer mittels Federelement 48 gefederten Halterung 47 gelagert, die nach Einführung der Trägervorrichtung 30 mit der Rohrwendel 20 aus **Fig. 5** und der Verbindung der Bodenplatte 36 mit dem Basisflansch 34 auf der Bodenplatte 36 zur Anlage kommt.

In dem Tauchrohr 11 ist eine Lampe 12 angeordnet, die die Einsatzstrahlung und ggf. auch Strahlung mit Wellenlängen, die von der Einsatzstrahlung abweichen, emittiert. Zur Kühlung der Lampe 12 und zur thermischen Abkopplung von dem Aufnahmeraum 38' weist das Lampenmodul 10 einen Tauchrohr-Zulaufanschluss 15 auf, über den der Tauchrohrinnenraum 11' mit einem weiteren flüssigen Temperiermedium K_{L} gefüllt werden kann, das wie das Tauchrohr 11 zumindest für die Einsatzstrahlung transparent gewählt wird. Zur Kreislaufführung des zweiten Temperiermediums K_{L} weist das Lampenmodul 10 ferner einen Tauchrohr-Ablaufanschluss 16 auf, mit dem das erwärmte Temperiermedium K_{L} über nicht dargestellte Kreislaufleitungen aus dem Tauchrohrinnenraum 11' abgeführt und nach Abgabe der aufgenommenen Wärme außerhalb des Schutzgehäuses 40 erneut über den Tauchrohr-Zulaufanschluss 15 zugeführt wird.

Zum Anschluss des Kühlkreislaufs und zum elektrischen Anschluss der Lampe 12 weist das Lampenmodul 10 ein Kopfteil 42 auf, das das Tauchrohr 11 am kopfseitigen Ende abdichtend verschließt. Das Kopfteil 42 aus **Fig. 1 bis 3** ist nicht direkt mit der Kopfplatte 39 verbunden, was aber durchaus der Fall sein kann, wie **Fig. 4 bis 5** zeigt: Dort ist das Kopfteil 42 mit der Kopfplatte 39 verbunden. Abweichend davon können Kopfplatte und Kopfteil auch integriert in einem Bauteil vorliegen (nicht dargestellt). **Fig. 1 bis 3** zeigen, dass sich der Tauchrohr-Zulaufanschluss 15 und der Tauchrohr-Ablaufanschluss 16 zur Verbindung des Tauchrohrinnenraums 11' mit einem Kühlkreislauf durch das Kopfteil 42 erstrecken, während das Kopfteil 42 in **Fig. 4** ein mit der Lampe 12 verbundenes elektrisches Anschlusselement 43 zeigt, das mit einem weiteren elektrischen Anschlusselement 43' zum Anschluss an eine externe Stromquelle verbunden ist. Die separate Darstellung der Kühlmittel- und Stromanschlüsse der Kopfteile 42 in **Fig. 1 bis 3** **und** **4** dient dabei nur der besseren Übersicht und stellt keinerlei Beschränkung dar. Vielmehr weist selbstverständlich auch das Lampenmodul 10 aus **Fig. 1 bis 3** entsprechende elektrische Anschlusselemente zur Energieversorgung der Lampe 12 auf, und das Lampenmodul 10 aus **Fig. 4** kann entsprechende Tauchrohr-Zulauf- und Tauchrohr-Ablaufanschlüsse für ein Kühlmittel K_{L} aufweisen.

Nicht dargestellt sind Schaltschränke und Vorschaltgeräte, die ein erfindungsgemäßer Wendel-Photoreaktor zur Steuerung der Lampe durch Leistungseinstellung (ggf. auch durch Pulsen der Lampe) umfassen kann, und die beispielsweise zur sicheren Abschaltung gemäß den ATEX-Richtlinien ausgebildet sein können.

Die Lampenmodule 10 in den gezeigten Wendel-Photoreaktoren 1 weisen als Strahlungsquelle eine LED-Lampe 12 mit mehreren LEDs 13 auf, die an einem Trägerkörper 14 über dessen Mantelfläche verteilt angeordnet sind. Eine LED-Lampe kann gegenüber herkömmlichen Strahlungsquellen auf Grund ihres vergleichsweise geringen Stromverbrauchs, hoher Lebensdauer und der hohen Schaltfestigkeit bei spontan vollem Lichtstrom bevorzugt eingesetzt werden. Die Einsatzstrahlung der LED-Lampe 13 kann durch geeignete Auswahl der LED 13 gezielt eingestellt werden, da die Wellenlänge der von LEDs emittierten Strahlung von der Dotierung des Halbleiterbauelements abhängt. LEDs sind zwar keine Wärmestrahler, allerdings verkürzen hohe Temperaturen, die im Betrieb in Abhängigkeit der Anordnung und der Leistung der LEDs durchaus auftreten, die Lebensdauer der LEDs deutlich. Um die dimmbaren LEDs für eine hohe Lichtausbeute bzw. Strahlungsintensität mit hohen Strömen zu betreiben, ist eine effektive Wärmeableitung erforderlich, um die Lebensdauer der LEDs zu erhalten.

Der Trägerkörper 14 kann daher zur Wärmeableitung aus einem Metall, insbesondere Aluminium, bestehen. Da diese Wärmeableitung beim Einsatz in Photoreaktoren für chemische Synthesen, die stark exotherm verlaufen können, häufig nicht ausreichend ist, erstreckt sich bei der dargestellten LED-Lampe 12 ein Fluidkanal 14' durch den Trägerkörper 14, der gleichzeitig als Kühlkörper fungiert, um Wärme, die der Trägerkörper 14 von den LEDs 13 aufgenommen hat, zumindest teilweise an das Kühlfluid K_{L} zu übertragen, das durch den Fluidkanal 14' strömt. Dazu ist der Fluidkanal 14' an einem kopfseitigen Ende des Trägerkörpers 14 mit dem Tauchrohr-Zulaufanschluss 15 verbunden, der sich durch das Kopfteil 42 erstreckt. In einer nicht dargestellten Variante ist es denkbar, dass der Verlauf des Fluidkanals innerhalb des Trägerkörpers eine Kehre am bodenseitigen Ende aufweist, sodass der Fluidkanal an dem kopfseitigen Ende des Trägerkörpers auch mit dem Tauchrohr-Ablaufabschluss verbunden sein kann.

Figurativ ist gezeigt, dass der Fluidkanal 14' erfindungsgemäß an dem bodenseitigen Ende des Trägerkörpers 14 durch eine Zulauföffnung 14" in den Tauchrohrinnenraum 11 mündet, sodass das am kopfseitigen Ende durch den Tauchrohr-Zulaufanschluss 15 zugeführte Kühlfluid K_{L} an dem bodenseitigen Ende des Trägerkörpers 14 austritt und an der Oberfläche der LED-Lampe 12 entlang zu dem kopfseitigen Ende des Lampenmoduls 10 strömt, wo es durch eine Ablauföffnung 16' an einer dem Tauchrohrinnenraum 11 zugewandten Seite des Kopfteils 42 in den Tauchrohr-Ablaufanschluss 16 gelangt, der sich parallel zu dem Tauchrohr-Zulaufanschluss 15 durch das Kopfteil 42 erstreckt. Nicht dargestellt sind Anschlussleitungen, die mit dem Tauchrohr-Zulauf- und Tauchrohr-Ablaufanschluss 15, 16 zum Ausbilden eines Kühlkreislaufs mit Pumpe und ggf. Wärmetauscher verbunden sind. So kann Wärme, die das Kühlfluid K_{L} durch den direkten Kontakt mit den LEDs 13 aufgenommen hat, außerhalb des Lampenmoduls 12 abgegeben werden. Durch Kreislaufführung des Kühlfluids K_{L} kann die Temperaturkontrolle der Lampe 12 unabhängig von einer Temperaturkontrolle des Reaktionsmediums in der Rohrwendel 20 erfolgen.

Da das Kühlfluid K_{L} die LEDs 13 und deren elektrische Anschlüsse direkt kontaktiert und sich in dem Bestrahlungsbereich der Lampe 12 befindet, wird als Kühlfluid K_{L} eine elektrisch nichtleitende, d. h. elektrisch isolierende Flüssigkeit gewählt, die für die Einsatzstrahlung transparent ist. So ist das Lampenmodul 10 hinsichtlich der Kühlung der LEDs 13 und der thermischen Abkopplung von dem Aufnahmeraum 38' verbessert und stellt darüber hinaus eine erhöhte Gesamtlicht- bzw. Strahlungsleistung, d. h. eine erhöhte Strahlungsmenge und -dichte an der Außenfläche des Tauchrohrs, in Bezug auf die Lampen gemäß Stand der Technik bereit, da die nichtleitende Flüssigkeit auf Grund eines Brechungsindex', der deutlich größer ist als der von Luft oder Inertgas und bei geeigneten nichtleitenden Flüssigkeiten im Bereich von etwa 1,35 bis etwa 1,55 (bei 20°C) liegt, eine erhöhte Photonen-Auskopplungseffizienz an der Phasengrenze Diodenoberfläche-Tauchrohrinnenraum und eine verringerte Reflexion an der Phasengrenze Tauchrohrinnenraum-Tauchrohrwand bereitstellt und so Nahfeldreflexionen vermeidet.

Ferner ist vorteilhaft, dass eine beschleunigte Alterung der Primäroptiken der LEDs vermieden wird, die gerade in Chemieanlagen, in denen VOCs ("volatile organic compounds") vorhanden sind, auch bei Verwendung von einem Inertgas wie Stickstoff auftreten können, da VOCs in die üblicherweise als Silikonlinse ausgeführten Primäroptiken eindringen, diese eintrüben und somit die Lichtausbeute senken. Da die Primäroptiken durch die nichtleitende Flüssigkeit von VOCs abgeschirmt werden, wird der Alterungsprozess deutlich verlangsamt.

Beispielsweise können als flüssiges Kühlmittel K_{L} dünnflüssige Silikonöle eingesetzt werden, die bis in den mittleren UV-C-Bereich transparent und nicht brennbar sind. Je nach Wellenlänge der Einsatzstrahlung können ggf. auch fluorierte Kohlenwasserstoffe wie Perfluorkohlenwasserstoffe und Hydrofluorether als Kühlmittel K_{L} eingesetzt werden, die vorteilhaft nicht brennbar sind, aber in gewissen Wellenlängenbereichen Absorptionsbanden aufweisen: Liegt die Einsatzstrahlung außerhalb der Absorptionsbanden können fluorierte Kohlenwasserstoffe wie beispielsweise 3M Fluorinert Electronic Liquid oder 3M Novec High-Tech Flüssigkeit von 3M^{™} (3M electronics, St. Paul, USA) eingesetzt werden.

Ferner kann, insbesondere im Spektralbereich unter 250 nm, auf hochraffinierte Mineralöle als Kühlmittel K_{L} zurückgegriffen werden, die hauptsächlich gesättigte Kohlenwasserstoffe umfassen. Vorteilhaft sind Alkane und Cycloalkane vom sichtbaren Wellenlängenbereich bis in den weiten UV-C-Bereich und bei ausreichend geringer Wegstrecke zwischen LED und Tauchrohr bis 195 nm und darunter transparent. Allerdings ist bei Verwendung von hochraffinierten Mineralölen als Kühlmittel auf einen sorgfältigen und abgedichteten Luftabschluss zu achten, um die Bildung entzündlicher Dampf-Luft-Gemische zu vermeiden. Weitere alternative Beispiele für ein Kühlmittel K_{L} umfassen synthetische Ester- und Etherverbindungen. Synthetische organische Esteröle, die bis in den mittleren UV-Bereich transparent sind, haben gegenüber Mineralölen den Vorteil u. a. einer höheren Temperaturbeständigkeit und höheren Brenn- und Zündtemperatur und sind umweltverträglicher, weisen aber eine geringere Alterungsbeständigkeit auf. Auch bei Etherverbindungen wie beispielsweise 1,4-Dioxan reicht die Transmission bis in den mittleren UV-Bereich, jedoch ist auch hier bei der Konstruktion des Lampenmoduls zur Vermeidung leicht entzündlicher Dampf-Luft-Gemische auf einen sorgfältigen Luftabschluss zu achten.

Sofern nicht bereits genannt, können die für das Lampenmodul 10 aufgezählten Temperiermedien K_{L} auch als Temperiermedium Ks zur Kühlung der Rohrwendel 20 gewählt werden. Als Temperiermedium Ks zur Kühlung der Rohrwendel 20 kann das gleiche Kühlmittel eingesetzt werden wie das Temperiermedium K_{L} zur Kühlung der Lampe 12, oder es können unterschiedliche Temperiermedien einsetzt werden. Vorzugsweise sind die beiden Kühlkreisläufe voneinander getrennt - je nach auftretenden Temperaturniveaus können beide Kühlkreisläufe ggf. aber auch miteinander verbunden sein.

Selbstverständlich kommen auch weitere Flüssigkeiten zum Einsatz als Kühlmittel K_{L} in Frage, solange sie elektrisch isolierend sind und für die Wellenlänge der Einsatzstrahlung transparent sind. Um eine für die gewünschte Transparenz erforderliche Transmission von zumindest 75 %, insbesondere bei Wellenlängen unter 250 nm, bereitzustellen, kann der Innendurchmesser des Tauchrohrs in Bezug auf den Außendurchmesser des mit den LEDs bestückten Trägerkörpers so gewählt werden, dass die Wegstrecke zwischen der LED-Oberfläche und der Tauchrohrinnenwand - und damit die Absorption durch das Kühlmittel - möglichst klein ist. Ferner ist bei der Auslegung in Bezug auf den Abstand zwischen Tauchrohr und Trägerkörper zu berücksichtigen, dass das Kühlmittel mit einem für einen optimalen Wärmeabtransport ausreichenden Volumenstrom und geeigneter Strömungsführung bereitgestellt wird.

Vorteilhaft ermöglich jeder erfindungsgemäße Wendel-Photoreaktor dadurch eine thermische Abkopplung und Temperierung der Lampe unabhängig von der Temperierung der Rohrwendel, d. h. des Reaktionsmediums bzw. Produktfluids mit dem Reaktionsprodukt. Dadurch ist es möglich, nicht nur stark exotherme Reaktionen mit großer Wärmeentwicklung, sondern u. a. auch Tieftemperaturreaktionen ohne Kondenswasserbildung im Tauchrohr durchzuführen. Ferner ist der Wendel-Photoreaktor durch die Trägervorrichtung flexibel anpassbar. Die Trägervorrichtung gestattet den Einsatz unterschiedlicher Rohrwendeln, die unterschiedliche, an die spektrale Absorption und Hydrodynamik (Plug-Flow) angepasste Durchmesser und/oder unterschiedliche Längen zur Anpassung der Verweilzeit in Korrelation mit dem Druckverlust in Abhängigkeit der Viskosität des Reaktions- bzw. Produktmediums aufweisen können. Ferner können sich mit der Trägervorrichtung einsetzbare Rohrwendeln hinsichtlich der Materialien unterscheiden, die je nach Reaktionsbedingungen angepasste Transmissionswerte und Druckfestigkeiten aufweisen können. Rohrwendeln aus festen Kunststoff- und Glasmaterialien ermöglichen auch kleineren Biegeradien, als durch Formung eines flexiblen Kunststoffschlauchs erreicht werden können. Außerdem können funktionalisierte Rohrwendeln mit immobilisierten Katalysatoren eingesetzt werden, die beispielsweise in einem Sol-Gel Verfahren fixiert sein können. Dazu kann eine katalysatorhaltige Beschichtungslösung (Sol) auf die Innenoberfläche appliziert werden, um nach Trocknung die gewünschte Beschichtung als Gelfilm mit vorzugsweise homogener, amorpher Struktur und gleichmäßiger, dünner Schichtdicke möglichst ohne Defekte zu erreichen, um Strahlungsverluste durch Reflexion bzw. Streuung an den Grenzflächen zu vermeiden. Rohrwendeln aus Quarzglas können beispielsweise mit einem anorganischen Gelfilm basierend auf SiO₂ versehen werden, der auch nach einer Verfestigungsbehandlung bei Temperaturen über 400 °C amorph bleibt. Die Beschichtungslösung enthält dabei zumindest ein photokatalytisches Material und ggf. weitere Metalloxide (z. B. Aluminium-, Titan- oder Yttriumoxid, ...), die die optischen Eigenschaften der Rohrwendel-Oberfläche beeinflussen können, ggf. aber auch photokatalytisch wirken können.

Ferner ermöglicht die separate Anordnung von Lampenmodul einerseits und Trägervorrichtung mit Rohrwendel andererseits im Schutzgehäuse auch einen einfachen Austausch des gesamten Lampenmoduls, um mit Einsatzstrahlungen unterschiedlicher Wellenlängen zu arbeiten. Ist das Tauchrohr in allen Wellenlängen der Einsatzstrahlungen transparent, ist es möglich, das Tauchrohr im Schutzgehäuse zu belassen und nur die Lampe auszutauschen, um die gewünschte Einsatzstrahlung bereitzustellen.

Im Gegensatz zu herkömmlichen chemischen Photoreaktoren, die meist für einen Batchbetrieb mit Tauchlampen ausgestattet sind, ist die Betriebssicherheit des kontinuierlich betriebenen Wendel-Photoreaktors erhöht, da sich nur vergleichsweise geringe Mengen des Reaktionsmediums in der Rohrwendel innerhalb des Schutzgehäuses befinden, das als Druckbehälter nach Druckgeräterichtlinien ausgelegt ist. Ferner eignet sich der erfindungsgemäße Wendel-Photoreaktor, der eine vereinfachte Skalierung von Labor auf Industriemaßstab zulässt, nicht nur zur Durchführung photochemischer Reaktionen in der Flüssigphase, bei denen ein flüssiges Eduktfluid in die Rohrwendel zugeführt wird, deren Windungen als flüssiges Reaktionsmedium passiert und die Rohrwendel als flüssiges Produktfluid mit dem Reaktionsprodukt verlässt, sondern auch zur Durchführung photochemischer Reaktionen in der Gasphase.

Die dargestellten Beispiele beziehen sich auf einen Wendel-Photoreaktor, der eine Rohrwendel aufweist, die um ein Lampenmodul angeordnet ist und von einer Trägervorrichtung gehalten wird, die zusammen in einem Schutzgehäuse angeordnet sind. Die Längsachse des Lampenmoduls ist hierbei identisch mit der Längsachse der Rohrwendel. In einer nicht dargestellten Modifikation kann ein erfindungsgemäßer Wendel-Photoreaktor auch mehrere Rohrwendeln aufweisen, die jeweils ein Lampenmodul umgeben und parallel in einem Schutzgehäuse angeordnet sind, wobei für jede Rohrwendel eine eigene Trägervorrichtung oder für alle Rohrwendeln eine gemeinsame Trägervorrichtung vorgesehen sein kann. Ebenfalls denkbar sind Modifikationen, bei der ein erfindungsgemäßer Wendel-Photoreaktor zwei (oder mehr) Rohrwendeln aufweist, die um ein Lampenmodul angeordnet sind, wobei die Windungen der Rohrwendeln die gleiche Steigung aufweisen und entsprechend einem zwei (oder mehr)gängigen Gewinde versetzt angeordnet sein können. Ferner können zwei oder mehr Lampenmodule parallel zur Längsachse einer Rohrwendel nebeneinander oder entlang der Längsachse hintereinander in einer Rohrwendel angeordnet werden, um die Strahlungsleistung zu erhöhen oder unterschiedliche photochemische Reaktionen mittels unterschiedlicher Wellenlängen der Einsatzstrahlung zu realisieren. Ferner kann ein erfindungsgemäßer Wendel-Photoreaktor in einer weiteren Ausführung zusätzliche Lampenmodule aufweisen, die in dem Schutzgehäuse außerhalb der Rohrwendel angeordnet sind, sodass die Rohrwendel nicht nur von innen, sondern auch von außen bestrahlt werden kann. Auch hier wird eine korrekte Positionierung der Rohrwendel in Bezug zu dem zusätzlichen Lampenmodul durch die Trägervorrichtung gesichert.

### BEZUGSZEICHENLISTE

- 1: Wendel-Photoreaktor
- 10: Lampenmodul
- 11, 11': Tauchrohr, Tauchrohrinnenraum
- 12: LED-Modul
- 13: LED
- 14, 14', 14": Träger-/Kühlkörper, Fluidkanal, Zulauföffnung
- 15: Tauchrohr-Zulaufanschluss
- 16, 16': Tauchrohr-Ablaufanschluss, Ablauföffnung
- 20: Rohrwendel
- 21: Eingangsabschnitt
- 22: Ausgangsabschnitt
- 23, 23a, 23b: Rohrwindung
- 24: Rücklaufleitung
- 30: Trägervorrichtung
- 31: Geführtes Eingriffselement
- 32: Halterungselement
- 33: Längliches Führungselement
- 34, 34': Basisflansch, Durchtrittsöffnung
- 35, 35': Bodenflansch, Durchtrittsöffnung
- 36: Bodenplatte
- 37, 37': Schutzgehäuse-Bodenabschnitt, Bodenraum
- 38, 38': Schutzgehäuse-Aufnahmeabschnitt, Aufnahmeraum
- 39: Kopfplatte
- 40: Schutzgehäuse
- 41, 41': Gehäuse-Zulaufanschluss, Gehäuse-Ablaufanschluss
- 42: Kopfteil
- 43, 43': Anschlusselement
- 44: Achselement
- 45: Befestigungsring
- 46: Befestigungsstab
- 47: Halterung
- 48: Federelement
- 50: Gestell
- 51: Rahmen
- 52: Befestigungsabschnitt

- K_{L}: Temperier- bzw. Kühlmittel (Lampenmodul)
- Ks: Temperier- bzw. Kühlmittel (Schutzgehäuse)
- E: Eduktfluid
- P: Produktfluid
- S: Schwenkachse

## Patentansprüche

1. Wendel-Photoreaktor (1) mit zumindest einem Lampenmodul (10) und mit zumindest einer Rohrwendel (20), die eine Mehrzahl Rohrwindungen (23, 23a, 23b) zwischen einem Eingangsabschnitt (21) und einem Ausgangsabschnitt (22) aufweist, wobei die zumindest eine Rohrwendel (20) um das zumindest eine Lampenmodul (10) angeordnet ist,
**dadurch gekennzeichnet, dass**
der Wendel-Photoreaktor (1) eine Trägervorrichtung (30), die die zumindest eine Rohrwendel (20) trägt, und ein Schutzgehäuse (40) das einen Aufnahmeraum (38') umgibt, aufweist, in dem die Trägervorrichtung (30) mit der zumindest einen Rohrwendel (20) und das zumindest eine Lampenmodul (10) angeordnet sind, wobei die Trägervorrichtung (30) eine vorbestimmte Positionierung der Rohrwendel (20) in Bezug zu dem zumindest einen Lampenmodul (10) und dem Schutzgehäuse (40) bereitstellt.

2. Wendel-Photoreaktor (1) nach Anspruch 1,
**dadurch gekennzeichnet, dass**
der Wendel-Photoreaktor (1) zumindest ein längliches Führungselement (33) aufweist, das parallel zu einer durch das Lampenmodul (10) definierten Längsachse in dem Schutzgehäuse (40) vorliegt, und
die Trägervorrichtung (30) zumindest ein Eingriffselement (31) aufweist, das an dem länglichen Führungselement (33) längsbeweglich führbar und positionierbar angeordnet ist, wobei
die Trägervorrichtung (30) zumindest ein Halterungselement (32) aufweist, das zum Halten zumindest eines Abschnitts der Rohrwendel (20) ausgebildet ist, wobei das zumindest eine Halterungselement (32) und das Eingriffselement (31) einstückig ausgebildet sind, oder wobei das zumindest eine Halterungselement (32) mit dem Eingriffselement (31) lösbar oder unlösbar verbunden ist.

3. Wendel-Photoreaktor (1) nach Anspruch 1 oder 2,
**dadurch gekennzeichnet, dass**
der Aufnahmeraum (38') abgedichtet ist und das Schutzgehäuse (40) einen Gehäuse-Zulaufanschluss (41) und einen Gehäuse-Ablaufanschluss (41') aufweist, sodass der Aufnahmeraum (38') mit einem ersten Temperiermedium (Ks) befüllbar ist, das bevorzugt ein flüssiges Temperiermedium (Ks) ist.

4. Wendel-Photoreaktor (1) nach zumindest einem der Ansprüche 1 bis 3,
**dadurch gekennzeichnet, dass**
das Schutzgehäuse (40) einen zylindrischen Aufnahmeabschnitt (38) aufweist, der
- einenends mit einer Kopfplatte (39) verbunden ist, an der das zumindest eine Lampenmodul (10) befestigt ist, und
- anderenends mit einem Gehäuseboden (37) oder mit einer Bodenplatte (36) verbunden ist.

5. Wendel-Photoreaktor (1) nach Anspruch 4,
**dadurch gekennzeichnet, dass**
der Gehäuse-Zulaufanschluss (41) an dem Gehäuseboden (37) oder benachbart zu der Bodenplatte (36) an dem zylindrischen Aufnahmeabschnitt (38) und
der Gehäuse-Ablaufanschluss (41') benachbart zu der Kopfplatte (39) an dem zylindrischen Aufnahmeabschnitt (38) angeordnet sind.

6. Wendel-Photoreaktor (1) nach Anspruch 4 oder 5,
**dadurch gekennzeichnet, dass**
der Eingangsabschnitt (21) und der Ausgangsabschnitt (22) auf derselben Seite der Rohrwendel (20) vorliegen, wobei entweder
- die Rohrwendel (20) als zweigängige Rohrwendel (20) ausgebildet ist, bei der sich an den Eingangsabschnitt (21) erste Windungen (23a) eines ersten Windungsgangs bis zu einer Kehrwindung (23c) anschließen, von der sich zweite Windungen (23b) eines zweiten Windungsgangs bis zu dem Ausgangsabschnitt (22) erstrecken, oder
- eine Rücklaufleitung (24) zwischen einem von dem Eingangsabschnitt (21) abgewandten Ende der Windungen (23) und dem Ausgangsabschnitt (22) angeordnet ist.

7. Wendel-Photoreaktor (1) nach zumindest einem der Ansprüche 1 bis 6,
**dadurch gekennzeichnet, dass**
das Lampenmodul (10) ein Tauchrohr (11) und zumindest eine Lampe (12) aufweist, die in dem Tauchrohr (11) angeordnet ist, wobei das Lampenmodul (10) einen Tauchrohr-Zulaufanschluss (15) und einen Tauchrohr-Ablaufanschluss (16) aufweist, die mit einem von dem Tauchrohr (11) begrenzten Tauchrohrinnenraum (11') kommunizieren, sodass der Tauchrohrinnenraum (11') mit einem zweiten Temperiermedium (K_{L}) befüllbar ist, das bevorzugt ein flüssiges Kühlmedium (K_{L}) ist.

8. Wendel-Photoreaktor (1) nach Anspruch 7,
**dadurch gekennzeichnet, dass**
das Lampenmodul (10) ein Kopfteil (42) mit zumindest einem elektrischen Anschlusselement (43') aufweist, das mit einem elektrischen Anschlusselement (43) der Lampe (10) verbunden ist, wobei das Kopfteil (42) und/oder die Kopfplatte (39), an der das Kopfteil (42) angeordnet ist, zur abgedichteten Halterung der Lampe (12) und/oder des Tauchrohrs (11) ausgebildet ist/sind, wobei sich der Tauchrohr-Zulaufanschluss (15) und der Tauchrohr-Ablaufanschluss (16) durch das Kopfteil (42) und/oder die Kopfplatte (39) erstrecken.

9. Wendel-Photoreaktor (1) nach Anspruch 7 oder 8,
**dadurch gekennzeichnet, dass**
die Lampe (12) eine LED-Lampe (12) ist, die eine Mehrzahl von LEDs (13) aufweist, die an einem Trägerkörper (14) über dessen Mantelfläche verteilt angeordnet sind, durch den sich ein Fluidkanal (14') erstreckt, der an einem kopfseitigen Ende mit dem Tauchrohr-Zulaufanschluss (15) verbunden ist und an einem bodenseitigen Ende des Trägerkörpers (14) durch eine Zulauföffnung (14") in den Tauchrohrinnenraum (11') mündet, der über eine Ablauföffnung (16') mit dem Tauchrohr-Ablaufanschluss (16) kommuniziert, die benachbart zu dem kopfseitigen Ende des Trägerkörpers (14) vorliegt.

10. Wendel-Photoreaktor (1) nach zumindest einem der Ansprüche 1 bis 9,
**dadurch gekennzeichnet, dass**
das Schutzgehäuse (40) um eine Schwenkachse (S), die in einem rechten Winkel zu einer Längsachse des Schutzgehäuses (40) verläuft, schwenkbar gelagert ist, sodass das Schutzgehäuse (40) mit der Trägervorrichtung (30), die die zumindest eine Rohrwendel (20) trägt, und dem zumindest einen Lampenmodul (10) von einer vertikalen Anordnung in eine horizontale Anordnung überführbar ist.
